# EUROPEAN PATENT APPLICATION

(11) **EP 4 763 991 A1**
(43) Date of publication of application: **24.06.2026**
(21) Application number: 23950290.9
(22) Date of filing: 01.09.2023
(51) Int. Cl.: C12P 19/56, C12N 9/10

(54) **METHOD FOR EFFICIENTLY PREPARING REBAUDIOSIDE M BY USING BIO-ENZYMATIC METHOD**

(30) Priority: 28.08.2023 CN 202311085268
(71) Applicant: Guilin Layn Natural Ingredients Corp., Guilin, Guangxi 541199 (CN); Hangzhou Synbiolab Biotechnology Co., Ltd., Hangzhou, Zhejiang 310000 (CN)
(72) Inventor: SONG, Yunfei, Guilin, Guangxi 541199 (CN); WANG, Xiao, Guilin, Guangxi 541199 (CN); XIE, Yongfu, Guilin, Guangxi 541199 (CN); LIU, Yanxue, Guilin, Guangxi 541199 (CN)
(74) Representative: Sun, Yiming
(86) International application number: PCT/CN2023/116557
(87) International publication number: WO 2025/043715

(57) **Abstract**

The present invention belongs to the technical field of biological catalytic conversion, and in particular, relates to methods for efficiently preparing rebaudioside M using a biological enzyme method. The method includes: adding rebaudioside A, uridine diphosphate glucose, glycosyltransferase and a salt into a buffer solution, mixing evenly to obtain a mixture, and allowing the mixture to undergo an enzyme-catalyzed reaction in a stirring state to obtain the rebaudioside M. The glycosyltransferase has an amino acid sequence as set forth in at least one of SEQ **ID** NOS. 1-12. The glycosyltransferase can also be coupled with a sucrose synthase to catalyze the rebaudioside A to produce the rebaudioside M in one step using sucrose as a glycosyl donor.

## Description

### TECHNICAL FIELD

The present invention belongs to the technical field of biological catalytic conversion, and in particular, relates to a method for efficiently preparing rebaudioside M using a biological enzyme method.

### DESCRIPTION OF RELATED ART

It has been discovered that a class of green and healthy natural sweeteners represented by steviol glycosides can be widely used as sugar substitutes. Steviol glycosides are natural sweeteners derived from the leaves of stevia rebaudiana (S. rebaudiana) by a plant extraction method. The origin of S. rebaudiana lies in the ancient forests of Paraguay and Brazil in South America. For hundreds of years, the local people have been grinding the leaves of S. rebaudiana and using them as a sweetener, the sweetness of which comes mainly from a mixture of various rebaudiosides contained in the leaves. Steviol glycosides, a mixture of glycosides formed by some diterpene glycoside compounds, has a chemical structure involving the condensation of a sugar ligand with a steviol aglycone. Glycosidic bonds in the steviol glycosides are largely indigestible, resulting in little energy release, which makes the steviol glycosides a highly attractive sugar substitute. Stevioside and rebaudioside A (RA), the most abundant components in the leaves of S. rebaudiana, are 200 times sweeter than sucrose and are the earliest sweeteners used commercially. However, the stevioside and rebaudioside A act on bitter taste receptors hTAS2R4 and hTAS2R14 in taste bunds, resulting in an obvious aftertaste of bitterness and poor palatability. This reduces people's acceptance of both stevioside and rebaudioside A, and affects their scope of application and market acceptance. Rebaudioside M (RM), which is present in the leaves of S. rebaudiana at a very low content (0.3%), offers a rapid and pure sweetness with extremely low calories. It is most similar to sucrose in terms of sensory properties, showing great significance for researches in the food and pharmaceutical industries. It is expected to replace sucrose, a traditional sugar source, and to be wildly used as a natural sweetener. Hence, Rebaudioside M is hailed as the next generation sweetener.

At present, the commercialized products of rebaudioside A have a sweetness quality inferior to that of rebaudioside M. However, the rebaudioside M with the best taste in S. rebaudiana is severely lacking in S. rebaudiana and has an extremely low natural content, making it impossible to commercially refine and purify the rebaudioside M directly from the leaves of S. rebaudiana in the same way as stevioside and rebaudioside A. As a result, the large-scale production and supply of rebaudioside M is limited. To improve the yield, the rebaudioside M can be produced by means of transgenic S. rebaudiana or microbial methods, which, however, should be performed based on a full understanding of the biosynthetic pathway, in particular a glycosylation process, of rebaudioside M.

At present, the biosynthetic pathway of steviol glycosides has been basically clarified, and a series of uridine diphosphate glycosyltransferases (UGTs) using uridine diphosphate glucose (UDP-glucose, UPPG) as a glycosyl donor have been identified. Based on the analysis of the biosynthetic pathway of rebaudioside M, it can be found that glycosylation in the direction of a C19-carboxyl group of rebaudioside A (where a β(1-2) glycosidic bond is formed) is a necessary step to produce rebaudioside M, and determines the natural content of rebaudioside M. The glycosylation of steviol glycosides depends on four UGTs in the cytoplasm of S. rebaudiana. These UGTs are all annotated as GT1 family members in the Carbohydrate Active Enzyme (CAZy) database (www.cazy.org), and take uridine diphosphate glucose (UDP-glucose) as the glycosyl donor. Glycosyltransferases UGT85C2 and UGT74G1 connect a first glucosyl group via a β-1 glycosidic bond at the positions of C13 hydroxyl and C19 carboxyl groups in the steviol aglycone, respectively; then, UGT91D2 adds a glycosyl group at C2 of the first glucosyl group via a β(1-2) glycosidic bond; and finally, UGT76G1 adds a glycosyl group at position C3 of the first glucosyl group. Zhang et al. found that S. rebaudiana itself lacks the ability to form a β(1-2) glycosidic bond in the direction of the C19-carboxyl group in the steviol glycosides, which is the direct cause of the low natural content of steviol glycosides, DM

At present, producing the rebaudioside M through a glycosylation reaction by taking rebaudioside A as a substrate typically requires a two-step conversion, in which rebaudioside A is first glycosylated to produce rebaudioside D, and then further glycosylated to produce rebaudioside M. This two-step catalytic method for producing rebaudioside M has the disadvantage of easily causing the accumulation of the intermediate products rebaudioside D, which is not conducive to subsequent separation and purification. Moreover, the two-step method for catalyzing rebaudioside A to produce rebaudioside M typically requires many enzymes, with cumbersome steps.

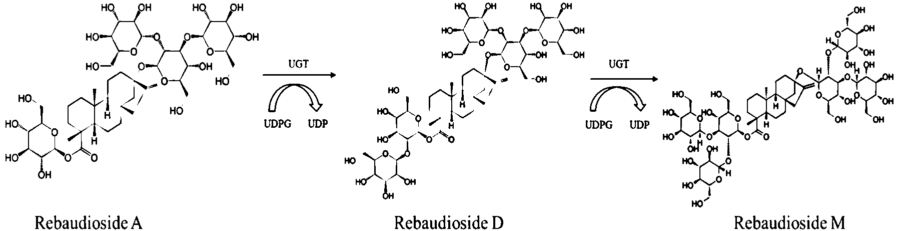

In general, the glycosylation reaction catalyzed by UDP-glycosyl donor-dependent glycosyltransferases (UGTs) requires the use of expensive and poor-stability UDP-glycosyl donors, such as UDP-glucose (UDPG). UDPG is too expensive, and its use as a glycosyl donor will incur huge costs and is not suitable for large-scale industrial production. As a result, the industrial application of UGTs is severely limited.

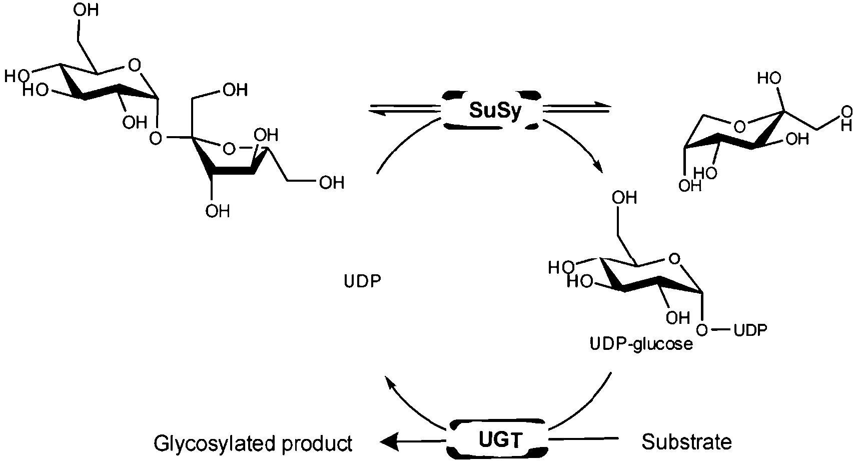

In an invention with Patent No.CN110734944B filed by Tianjin University and entitled "Method for One-step Synthesis of Rebaudioside M", with rebaudioside A as a substrate, rebaudioside M is obtained by adding uridine diphosphate glucose, magnesium sulfate or magnesium chloride, and methanol, and catalyzing the reaction using recombinant engineered bacteria UGT1 and UGT2 capable of secreting and expressing glycosyltransferases. In this method, a large amount of metal ions and organic solvents are added, which is not conducive to subsequent separation, purification and food-grad production. Moreover, the addition of expensive uridine diphosphate glucose for the reaction leads to excessively high production costs. In an application with Patent No. CN109750071A filed by Nanjing Universality of Technology and entitled "Method for Biocatalytic Synthesis of Rebaudioside M", tomato-derived UDP-glycosyltransferases and potato-derived sucrose synthases are used to synthesize rebaudioside E by a glycosylation reaction using steviol glycosides as a starting material; and then, S. rebaudiana-derived UDP-glycosyltransferases and potato-derived sucrose synthases are used to synthesize rebaudioside M by a further glycosylation reaction using rebaudioside E as a starting material. This method requires the prior production of rebaudioside E before the further production of rebaudioside M. This method requires two or more steps of catalytic reactions to obtain a final product, resulting in slow reaction speed, low catalytic efficiency, and numerous by-products, which is not conducive to separation and purification.

Hence, current techniques for producing rebaudioside M have the following shortcomings:
1) the plant extraction method has complicated steps, low yield, high cost and difficulty in separation and purification;
2) the plant extraction method requires a large amount of S. rebaudiana, causing wastes of arable land;
3) the chemical synthesis method leads to a large amount of harmful gases and waste liquids, which is not conducive to environmental production;
4) the catalytic reaction system contains many metal ions and organic solvents, which is not conducive to subsequent separation, purification and food-grade production;
5) two or more steps of catalytic reaction are required to obtain the final product, leading to slow reaction speed, low catalytic efficiency and many by-products, which is not conducive to separation and purification;
6) uridine diphosphate glucose, as the commonly required sugar donor, is expensive, leading to high production costs, making it unsuitable for large-scale industrial production; and
7) a variety of enzyme preparations need to be added to catalyze the reaction, leading to poor specificity and low yield.

Therefore, there is an urgent need for a high-efficiency and low-cost method for producing rebaudioside M.

### BRIEF SUMMARY OF THE INVENTION

In order to overcome the shortcomings of the prior art, an object of the present invention is to provide a method for efficiently preparing rebaudioside M using a biological enzymatic method.

The method provided by the present invention involves obtaining a glycosyltransferase based on a gene mining method, then constructing engineered bacteria for protein expression, and performing a glycosylation reaction under in vitro catalysis using rebaudioside A as a substrate, thereby synthesizing rebaudioside M in one step. This method has high catalytic efficiency and short reaction time, and allows for producing a single product that is easy to separate, showing great industrial application prospects. Moreover, the glycosyltransferase provided by the present invention may also be coupled with a sucrose synthase to catalyze the rebaudioside A to produce the rebaudioside M in one step by taking sucrose as a glycosyl donor.

According to the method for efficiently preparing rebaudioside M using a biological enzymatic method provided by the present invention, by using the glycosyltransferase with high catalytic efficiency, the rebaudioside M can be produced through one-step catalysis using the rebaudioside A as a substrate, thereby enabling efficient biosynthesis of the rebaudioside M. This method can avoid the formation of an intermediate product rebaudioside D, thereby facilitating subsequent separation and purification and streamlining the process flow of the catalytic reaction. In this method, the biological enzymatic method is used to efficiently synthesize and prepare the natural sweetener rebaudioside M with high sweetness and low calories.

The object of the present invention is at least achieved by means of one of the following technical solutions.

The present invention provides a method for efficiently preparing rebaudioside M using a biological enzyme method, including the steps of:
adding rebaudioside A, uridine diphosphate glucose, glycosyltransferase and a salt into a buffer solution, mixing evenly to obtain a mixture, and allowing the mixture to undergo an enzyme-catalyzed reaction in a stirring state to obtain the rebaudioside M.

Further, in the mixture, the rebaudioside A has a concentration of 1 g/L to 100 g/L; and
preferably, in the mixture, the rebaudioside A has a concentration of 1 g/L.

Further, in the mixture, the uridine diphosphate glucose has a concentration of 0.5 mM to 20 mM; and
preferably, in the mixture, the uridine diphosphate glucose has a concentration of 1 mM.

Further, the salt is at least one of a calcium salt, a cobalt salt, an iron salt, a magnesium salt, a manganese salt, an ammonium salt, a nickel salt and a zinc salt;
preferably, the salt is at least one of a calcium salt, a magnesium salt, a manganese salt and an iron salt; and
further preferably, the salt is a magnesium salt.

Further, in the mixture, the salt has a concentration of 0.1 mM to 10 mM; and
preferably, in the mixture, the salt has a concentration of 1 mM.

Further, the buffer solution has a pH of 5.0 to 9.0;
preferably, the buffer solution has a pH of 6.0 to 8.0;
further preferably, the buffer solution has a pH of 8.0;
further, in the stirring state, a stirring speed is 50 rpm to 220 rpm; and
preferably, in the stirring state, a stirring speed is 200 rpm.

Further, the enzyme-catalyzed reaction occurs at a temperature of 20°C to 45°C for a duration of 1 h to 5 h.

Preferably, the enzyme-catalyzed reaction occurs at a temperature of 30°C to 37°C.

Further preferably, the enzyme-catalyzed reaction occurs at a temperature of 37°C

Further, in the mixture, the glycosyltransferase has a concentration of 1 g/L to 20 g/L; and
preferably, in the mixture, the glycosyltransferase has a concentration of 5 g/L.

Further, a sequence of the glycosyltransferase is at least one of amino acid sequences as set forth in SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 and SEQ ID NO. 12.

Preferably, the glycosyltransferase has an amino acid sequence as set forth in SEQ ID NO. 1.

In the method provided by the present invention, the glycosyltransferase used has high efficiency in catalyzing the glycosylation reaction, the uridine diphosphate glucose (UDPG) is used as a glycosyl donor, and this glycosyltransferase can effectively catalyze the glycosylation of rebaudioside A to produce the high-value sweetener rebaudioside M in one step, without the addition of any other enzymes.

In the method provided by the present invention, the optimal pH for the glycosylation reaction using the glycosyltransferase is 8.0, and the optimal reaction temperature is 37°C. The glycosyltransferase is a metal ion-dependent enzyme, and Mg²⁺, Ca²⁺ or Mn²⁺ shows a significant effect of promoting the catalytic efficiency of the glycosyltransferase, with Mg²⁺ exhibiting the strongest promoting effect.

In the method provided by the present invention, the optimal reaction conditions for the glycosyltransferase are as follows: the final concentrations of respective substances in a reaction system are respectively as follows: rebaudioside A: 1 g/L, UDPG: 1 mM, MgCl₂: 5 mM; the reaction temperature is 37°C; the revolving speed is 220 rpm; the reaction time is 1 h to 2 h. Under the optimal reaction conditions, the glycosyltransferase exhibits high catalytic efficiency in catalyzing the glycosylation of the rebaudioside A to produce the rebaudioside M, with the conversion rate up to more than 95%.

The present invention provides another method for efficiently preparing rebaudioside M using a biological enzyme method, including the steps of:
adding rebaudioside A, sucrose, UDP (5-uridine diphosphate disodium salt), glycosyltransferase, sucrose synthetase and a salt into a buffer solution, mixing evenly to obtain a mixture, and allowing the mixture to undergo an enzyme-catalyzed reaction in a stirring state to obtain the rebaudioside M.

Further, in the mixture, the rebaudioside A has a concentration of 1 g/L to 100 g/L; and
preferably, in the mixture, the rebaudioside A has a concentration of 10 g/L.

Further, in the mixture, the sucrose has a concentration of 1 mM to 500 mM; and
preferably, in the mixture, the sucrose has a concentration of 100 mM.

Further, in the mixture, the UDP has a concentration of 0.1 mM to 20 mM; and
preferably, in the mixture, the UDP has a concentration of 2 mM.

Further, the salt is at least one of a calcium salt, a cobalt salt, an iron salt, a magnesium salt, a manganese salt, an ammonium salt, a nickel salt and a zinc salt;
preferably, the salt is at least one of a calcium salt, a magnesium salt, a manganese salt and an iron salt; and
Further, in the mixture, the salt has a concentration of 0.1 mM to 10 mM; and
preferably, in the mixture, the salt has a concentration of 0.1 mM to 5 mM.

Further preferably, in the mixture, the salt has a concentration of 1 mM.

Further, the buffer solution has a pH of 4.0 to 9.0;
preferably, the buffer solution has a pH of 6.0 to 8.0;
further preferably, the buffer solution has a pH of 8.0;
further, in the stirring state, a stirring speed is 50 rpm to 220 rpm; and
preferably, in the stirring state, a stirring speed is 200 rpm.

Further, the enzyme-catalyzed reaction occurs at a temperature of 20°C to 45°C for a duration of 1 h to 5 h.

Preferably, the enzyme-catalyzed reaction occurs at a temperature of 30°C to 37°C.

Further preferably, the enzyme-catalyzed reaction occurs at a temperature of 37°C

Further, in the mixture, the glycosyltransferase has a concentration of 1 g/L to 20 g/L; and
preferably, in the mixture, the glycosyltransferase has a concentration of 5 g/L.

Further, a sequence of the glycosyltransferase is at least one of amino acid sequences as set forth in SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 and SEQ ID NO. 12.

Preferably, the glycosyltransferase has an amino acid sequence as set forth in SEQ ID NO. 1.

Further, in the mixture, the sucrose synthetase has a concentration of 1 g/L to 10 g/L; and
preferably, in the mixture, the sucrose synthetase has a concentration of 2 g/L.

Further, the sucrose synthetase has an amino acid sequence as set forth in at least one of SEQ ID NO. 13, SEQ ID NO. 14, SEQ ID NO. 15, SEQ ID NO. 16, SEQ ID NO. 17, SEQ ID NO. 18, SEQ ID NO. 19 and SEQ ID NO. 20.

Preferably, the sucrose synthetase has an amino acid sequence as set forth in SEQ ID NO. 13.

In the second method for efficiently preparing rebaudioside M using a biological enzyme method provided by the present invention, the sucrose is used as a glycosyl donor, and the glycosyltransferase may be coupled with the sucrose synthetase for catalysis in the presence of UDP, allowing for producing the high-value sweetener rebaudioside M from rebaudioside A in one step, without the production of any other intermediate products or by-products. In this method, the optimal pH for the double-enzyme coupled catalytic reaction is 8.0, and the optimal reaction temperature is 37°C. Mg²⁺ shows a significant effect of promoting the catalytic efficiency of the coupling catalytic reaction. In this method, the duration of the double-enzyme coupled reaction is short, and all the rebaudioside A is converted into rebaudioside M within 2 h.

In this method, the coupled catalysis from the glycosyltransferase and the sucrose synthase is utilized, and the sucrose is used as the glycosyl donor to replace the expensive UDPG to efficiently catalyze RA to produce RM in one step, thereby reducing production costs.

In both of the two methods provided by the present invention, the glycosyltransferase with high catalytic efficiency is used, allowing for producing the rebaudioside M through one-step catalysis using the rebaudioside A as a substrate, thereby enabling efficient biosynthesis of the rebaudioside M. These two methods can reduce metal ions, by-products and other impurities in the system and reduce the cost of separation and purification, enabling green biosynthesis of the rebaudioside M.

In the second method provided by the present invention, the cheap sucrose is used as the glycosyl donor, leading to greatly reduced production costs.

Compared with the prior art, the present invention has the following advantages and beneficial effects.
(1) In the methods provided by the embodiments of the present invention, the enzyme used can efficiently catalyze the rebaudioside A to produce the rebaudioside M in one step under optimal reaction conditions; no other enzymes are needed during a catalytic process, such that the glycosylation reaction can be specifically catalyzed to produce the rebaudioside M, without intermediate products or other by-products; and the reaction time is short, the catalytic efficiency is high, and the yield of the product rebaudioside M can reach more than 95%, which is conducive to the separation and purification of the product.
(2) In the methods provided by the embodiments of the present invention, the rebaudioside A can be catalyzed to produce the rebaudioside M in one step under optimal reaction conditions by means of double-enzyme coupled catalysis, without the production of by-products and with good specificity, which is conducive to subsequent product separation and purification; and the use of the sucrose as the glycosyl donor leads to reduced production costs, which is conducive to large-scale biosynthesis of rebaudioside M.
(3) In the methods provided by the embodiments of the present invention, the final reaction system contains only trace amounts of metal ions (Mg²⁺), without any intermediate products or by-products, and the impurities are extremely low in content and easy to separate, which is conducive to subsequent separation and purification in industrial-scale production.

### BRIEF DESCRIPTION OF THE SEVERAL VIEWS OF THE DRAWINGS

FIG. 1 is a graph showing the catalytic efficiency results of glycosyltransferase at different pH;
FIG. 2 is a graph showing the catalytic efficiency results of glycosyltransferase at different temperatures;
FIG. 3 is a graph showing the results of effects of different metal ions on the catalytic efficiency of glycosyltransferase;
FIG. 4 is a liquid chromatography chart showing the synthesis of rebaudioside M from rebaudioside A under catalysis using UDPG as a glycosyl donor; and
FIG. 5 is a liquid chromatography chart showing the synthesis of rebaudioside M from rebaudioside A under catalysis using sucrose as a glycosyl donor.

### DETAILED DESCRIPTION OF THE INVENTION

The specific implementation of the present invention will be further illustrated below in conjunction with examples, but the implementation and protection of the present invention are not limited thereto. It should be noted that any process that is not specifically described in detail below can be implemented or understood by those skilled in the art with reference to the prior art. Reagents or instruments as used without clearly indicating manufacturers are considered as commercially available conventional products.

### Example 1: Gene Mining

First, by taking UGT genes as probe sequences, homology searches were carried out to obtain several homologous sequences; multiple sequence alignment was carried out with mega software (MEGA-X); then, an evolutionary tree was constructed based on the sequences from which conserved sites were extracted, where the evolutionary tree can visually display the evolutionary relationship among the respective homologous sequences; several corresponding enzyme protein gene sequences that were close to the probe sequence species were selected as potential candidate genes; next, molecular substrate docking was performed through Rosetta homology modeling; free energy, binding energy and the like were calculated; and candidate gene sequences were further screened for subsequent functional verification.

### Example 2: Construction of Recombinant Plasmids

The sequence and codon of each screened glycosyltransferase gene were optimized, to synthesize a fragment of the above screened glycosyltransferase gene (6 his tags were added to the C-termini of the fragment during the synthesis process to facilitate the subsequent separation and purification of a recombinant enzyme using an Ni²⁺ affinity chromatography column), and then the recombinant gene fragment was attached to an E. *coli* expression vector pET28a using restriction endonucleases BamH I and EcoR I, thereby obtaining a recombinant plasmid carrying a target gene.

Recombinant *E*. *coli* was constructed with a heat shock transformation method. Several synthesized recombinant plasmids were transferred into *E*. *coli* DH5α (for PCR amplification of the recombinant plasmids) and *E*. *coli* BL21(DE3) (for heterologous expression proteins of recombinase) competent cells, respectively, to obtain the corresponding recombinant *E*. *coli* containing the target gene.

### Example 3: Expression of Recombinase

(1) Activated culture: 5 µL of glycerol bacteria was inoculated into 5 mL of LB liquid medium containing Kan resistance genes (final concentration: 100 µg/mL), and placed in a biochemical incubator at 37°C and 220 r/min for overnight culture (for 16 hours).
(2) Extended culture: 8 mL of an activated bacterial solution was transferred to a 1 L Erlenmeyer flask containing 800 mL of 2 × YT liquid medium (containing Kan resistance genes) using a pipette, and cultured in a biochemical incubator at 37°C and 220 r/min for a certain period of time, while the OD value was monitored.
(3) Induced expression: When the OD value of the bacterial solution was 0.6 to 0.8, the culture at 37°C was stopped 400µL of IPTG (0.5 mM) inducer was added to the Erlenmeyer flask, the temperature was changed to 16°C, the rotation speed was kept unchanged, and the culture was proceeded at low temperature in a constant-temperature shaking incubator for 16 h to 20 h to induce overexpression of the recombinant enzyme.
(4) Centrifugal bacterial collection: After low-temperature induced expression in step (3), a recombinant enzyme bacterial solution was centrifuged using a floor-standing centrifuge, with the temperature set to 4°C, the rotation speed set to 6000 rpm, and the time set to 10 min; and after the centrifugation is completed, a supernatant was discarded, leaving a bacterial sediment. The bacterial sediment was resuspended and washed with 50 mM PBS buffer (pH = 8.0) to 40 mL, and then centrifuged at 4°C and 8000 rpm for 10 min, the supernatant was discarded, and bacterial pellets were collected.
(5) Bacteria disruption: After the above washing, 50 mL of 50 mM PBS buffer (pH = 8.0) was added to the bacterial pellets for resuspending, and then, cells were disrupted under high pressure using a high-pressure and low-temperature disruptor. Centrifuging was performed at 8000 rpm for 30 min, and a supernatant is a crude enzyme solution.
(6) Measurement of protein concentration of crude enzyme solution: The total protein content in the crude enzyme solution was measured in a multifunctional microplate reader using a Pierce BCA protein quantification kit.

### Example 4: Univariate Factor Investigation of Glycosyltransferases

A univariate factor investigation experiment was carried out on the glycosyltransferase (as set forth in SEQ ID NO. 1) under enzyme-catalyzed reaction conditions to identify the enzyme properties of the glycosyltransferase.
1) To investigate the effect of the pH of a reaction system on the catalytic efficiency of the glycosyltransferase, three buffer systems, including an acetate buffer solution (50 mM, with a pH buffer range of 4.0 to 5.5), a phosphate buffer solution (50 mM, with a pH buffer range of 6.0 to 8.0) and a tris(hydroxymethyl)aminomethane hydrochloride buffer solution (50 mM, with a pH buffer range of 7.5 to 9.0), were used to subsequently prepare three corresponding reaction systems (see Table 1) at 32.5°C, and enzyme activity changes were detected within the range of pH 4.0 to 9.0.

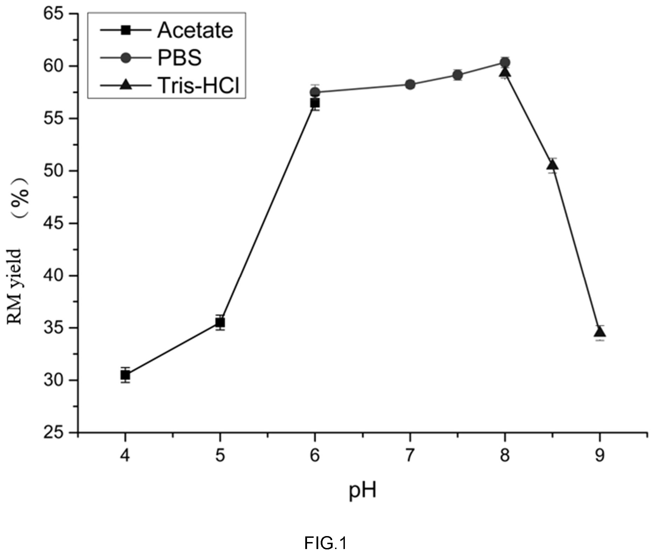

As shown in FIG. 1, the optimal pH for the glycosyltransferase is 8.0, and when pH is equal to 6.0, the glycosyltransferase maintains 65% of the catalytic efficiency, indicating that the glycosyltransferase has certain potential for large-scale biocatalytic production of rebaudioside M. The optimal pH and catalytic efficiency of other amino acid sequences as set forth in SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 and SEQ ID NO. 12 are similar to those of the amino acid sequence as set forth in SEQ ID NO. 1, as shown in FIG. 1.

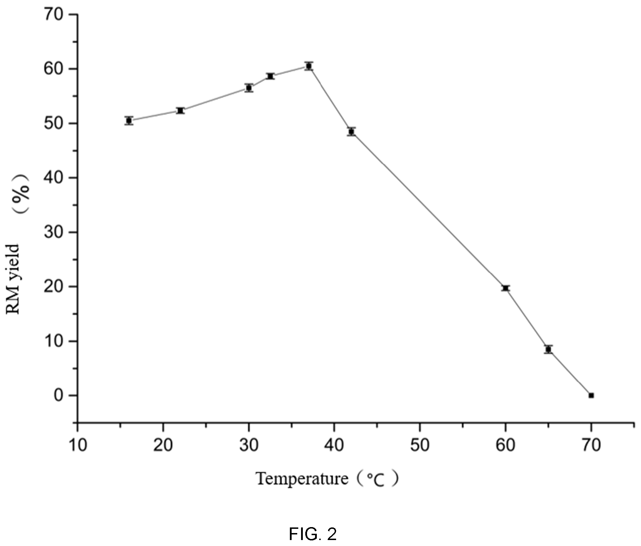

2) To investigate the effect of temperature on the catalytic efficiency of the glycosyltransferase, the catalytic efficiency of the glycosyltransferase was measured at pH 8.0 at 16°C, 22°C, 30°C, 32.5°C, 37°C, 42°C, and 65°C, respectively. This glycosyltransferase significantly decreased in catalytic efficiency at temperatures above 40°C, and exhibited a high catalytic activity within the range of 30°C to 37°C (as shown in FIG. 2).

As shown in FIG. 2, the yield of rebaudioside M is highest at 37°C. Therefore, this glycosyltransferase (and all glycosyltransferases show the same trend) exhibits the highest catalytic efficiency at 37°C. The optimal temperature and catalytic efficiency of other amino acid sequences as set forth in SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 and SEQ ID NO. 12 are similar to those of the amino acid sequence as set forth in SEQ ID NO. 1, as shown in FIG. 2.

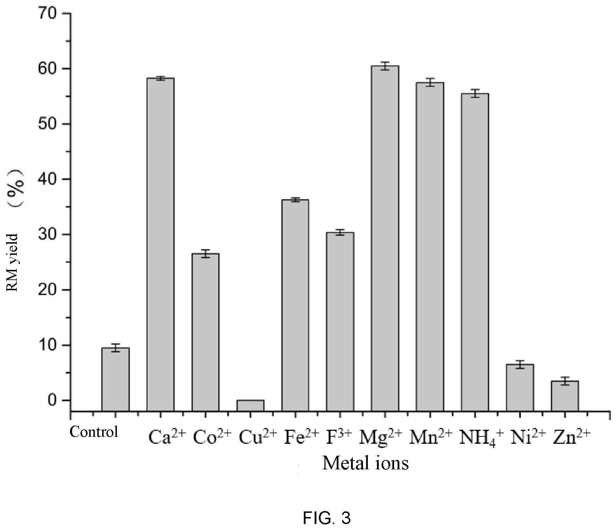

3) This experiment investigates the effects of various metal ions and ammonium ions on the catalytic efficiency of glycosyltransferases. As can be seen from FIG. 3, Mg²⁺, Ca²⁺, Mn²⁺ and Fe³⁺ show a significant effect of promoting the catalytic efficiency of the glycosyltransferase. Here, Mg²⁺ exhibits the strongest promoting effect, and under the same conditions, the yield of rebaudioside M can reach 61%, which is approximately 6 times higher than that of a blank control group; and in the presence of Ca²⁺ and Mn²⁺, the yield of rebaudioside M is approximately 56%. It should be noted that NH⁴⁺ also has a strong promoting effect on this glycosyltransferase, with the yield of rebaudioside M being 55%. Cu²⁺, Zn²⁺ and Ni²⁺ have a significant inhibitory effect on the catalytic efficiency of this glycosyltransferase. Here, Cu²⁺ ions exhibits the strongest inhibitory effect on this glycosyltransferase, and in the presence of copper ions, this glycosyltransferase loses activity, with the yield of rebaudioside M being 0; and in the presence of Zn²⁺, the catalytic efficiency of this glycosyltransferase is only half that of the blank control group.

The above results indicate that this glycosyltransferase is a metal-dependent enzyme, with Mg²⁺ exhibiting the strongest promoting effect on this glycosyltransferase and Cu2+ exhibiting the strongest inhibitory effect on this glycosyltransferase, which can directly destroy the enzymic protein structure and thereby inactivate the glycosyltransferase.

The effects of different metal ions and ammonium ions on the catalytic efficiency of the amino acid sequences as set forth in SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 and SEQ ID NO. 12 are similar to that of the amino acid sequence as set forth in SEQ ID NO. 1, as shown in FIG. 3.

Example 5: Synthesis of Rebaudioside M from Rebaudioside A under Double-enzymatic Catalysis using Sucrose as Glycosyl Donor

### 1) Catalytic reaction system

1mM UDPG, a crude glycosyltransferase solution (approximately 3 mg/mL total protein), 1g/L rebaudioside A, and 5 mM magnesium chloride were added to a reaction system, and PBS (50 mM) at the pH of 8.0 was supplemented till reaching a final reaction volume. A reaction was allowed to proceed for 2 h at 37°C and 200 rpm; a sample was taken, placed in a water bath at 100°C, heated for 10 min, and centrifuged at 12,000 rpm for 1 min; a supernatant was filtered through a 0.45 µm sterile filter membrane into a liquid phase vial; and a liquid phase was tested and analyzed by HPLC.

### 2) High-performance liquid chromatography (HPLC) test method

The contents of substrates and products in a reaction system after the enzymatic reaction are tested by HPLC. column Model: Inertsil^{™} ODS-3 reverse-phase C18 column; column temperature: 40°C; detector: PDA (ultraviolet) detector; detection wavelength: 218 nm; mobile phase A: acetonitrile containing 0.1% formic acid; mobile phase C: ultrapure water containing 0.1% formic acid; %: volume percentage; liquid-phase gradient elution procedures: 0 min (25% A, 75% C), 4 min (40% A, 60% C), 15 min (42% A, 58% C), 16 min (100% A, 0% C), 21 min (5% A, 95%C), 30 min (25% A, 75% C); flow velocity: 0.6 mL/min; injection volume: 10 µL.

### 3) Results

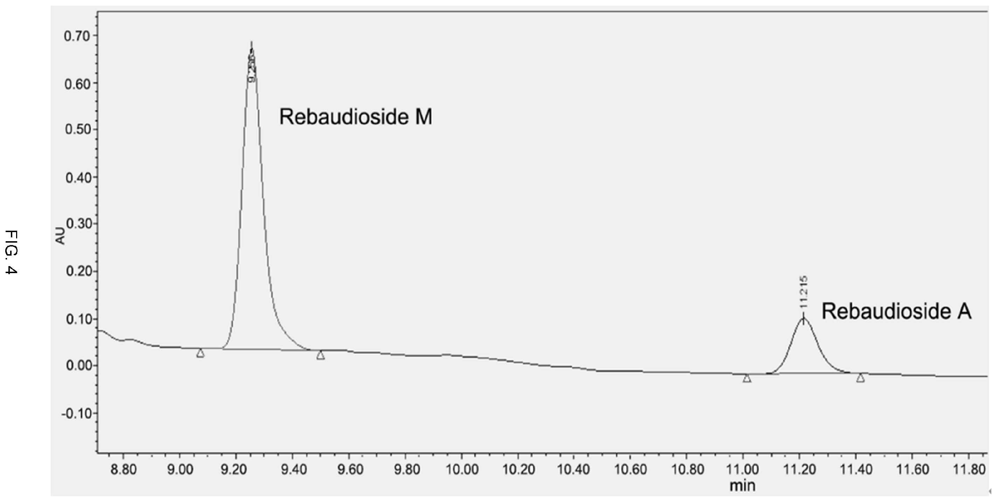

As shown in FIG. 4, based on the analysis and calculation from the HPLC results, after 2 hours of reaction, the rebaudioside A is almost completely converted into rebaudioside M, with a conversion rate up to 98% and without any by-products produced.

Example 6: Synthesis of Rebaudioside M from Rebaudioside A under Enzymic Catalysis using UDPG as Glycosyl Donor

### 1) Catalytic reaction system

34 g/L sucrose, 10 g/L rebaudioside A, a crude glycosyltransferase solution (approximately 5 mg/mL total protein), a crude sucrose synthase solution (approximately 2 mg/mL total protein), 2 mM uridine diphosphate disodium salt, and 5 mM magnesium chloride were added to a reaction system, and PBS (50 mM) at the pH of 8.0 was supplemented till reaching a final reaction volume. A reaction was allowed to proceed for 3 h at 37°C and 200 rpm; a sample was taken, placed in a water bath at 100°C, heated for 10 min, and centrifuged at 12,000 rpm for 1 min; a supernatant was filtered through a 0.45 µm sterile filter membrane into a liquid phase vial; and a liquid phase was tested and analyzed by HPLC.

### 2) HPLC test method

The contents of substrates and products in a reaction system after the enzymatic reaction are tested by HPLC. column Model: Inertsil^{™} ODS-3 reverse-phase C18 column; column temperature: 40°C; detector: PDA (ultraviolet) detector; detection wavelength: 218 nm; mobile phase A: acetonitrile containing 0.1% formic acid; mobile phase C: ultrapure water containing 0.1% formic acid; %: volume percentage; liquid-phase gradient elution procedures: 0 min (25% A, 75% C), 4 min (40% A, 60% C), 15 min (42% A, 58% C), 16 min (100% A, 0% C), 21 min (5% A, 95%C), 30 min (25% A, 75% C); flow velocity: 0.6 mL/min; injection volume: 10 µL.

### 3) Results

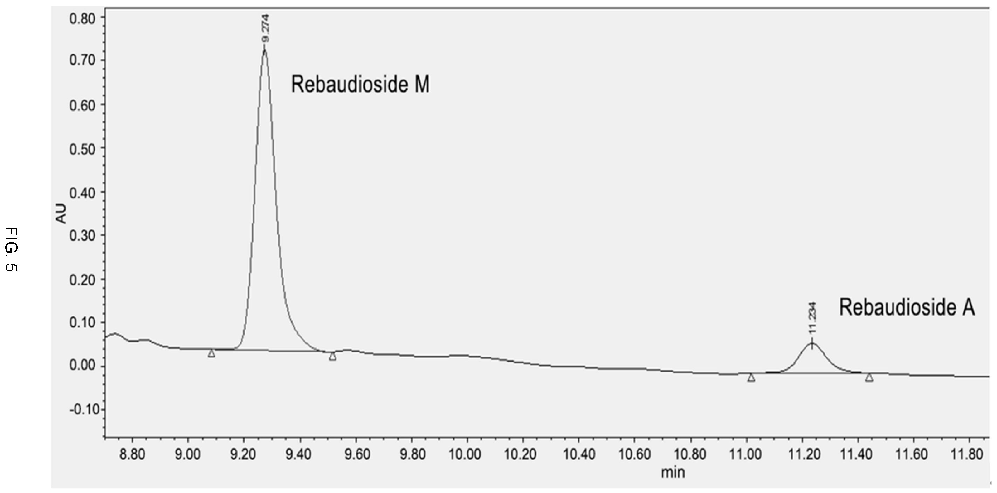

As shown in FIG. 5, based on the analysis and calculation from the HPLC results, after 3 hours of double-enzymatic catalytic reaction of the rebaudioside A with sucrose as the glycosyl donor, the rebaudioside A is almost completely converted into rebaudioside M, with a conversion rate up to 95% and without any by-products produced.

The above embodiments are only preferred embodiments of the present invention, and are only intended to illustrate the present invention, rather than limiting the present invention. Any variation, substitution, modification and the like made by those skilled in the art without departing from the spirit and essence of the present invention should fall within the scope of protection of the present invention.

## Claims

1. A method for efficiently preparing rebaudioside M using a biological enzyme method, comprising the steps of:
adding rebaudioside A, uridine diphosphate glucose, glycosyltransferase and a salt into a buffer solution, mixing evenly to obtain a mixture, and allowing the mixture to undergo an enzyme-catalyzed reaction in a stirring state to obtain the rebaudioside M.

2. The method for efficiently preparing rebaudioside M using the biological enzyme method according to claim 1, wherein in the mixture, the rebaudioside A has a concentration of 1 g/L to 100 g/L; in the mixture, the uridine diphosphate glucose has a concentration of 0.5 mM to 20 mM; the salt is at least one of a calcium salt, a cobalt salt, an iron salt, a magnesium salt, a manganese salt, an ammonium salt, a nickel salt and a zinc salt; and in the mixture, the salt has a concentration of 1 mM to 10 mM.

3. The method for efficiently preparing rebaudioside M using the biological enzyme method according to claim 2, wherein the salt is at least one of a calcium salt, a magnesium salt, a manganese salt and an iron salt; and in the mixture, the salt has a concentration of 0.1 mM to 5 mM.

4. The method for efficiently preparing rebaudioside M using the biological enzyme method according to claim 1, wherein in the mixture, the buffer solution has a pH of 5.0 to 9.0; in the stirring state, a stirring speed is 50 rpm to 220 rpm; and the enzyme-catalyzed reaction occurs at a temperature of 20°C to 45°C for a duration of 1 h to 5 h.

5. The method for efficiently preparing rebaudioside M using the biological enzyme method according to claim 1, wherein in the mixture, the glycosyltransferase has a concentration of 0.1 g/L to 20 g/L; and a sequence of the glycosyltransferase is at least one of amino acid sequences as set forth in SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 and SEQ ID NO. 12.

6. A method for efficiently preparing rebaudioside M using a biological enzyme method, comprising the steps of:
adding rebaudioside A, sucrose, 5-uridine diphosphate disodium salt, glycosyltransferase, sucrose synthetase and a salt into a buffer solution, mixing evenly to obtain a mixture, and allowing the mixture to undergo an enzyme-catalyzed reaction in a stirring state to obtain the rebaudioside M.

7. The method for efficiently preparing rebaudioside M using the biological enzyme method according to claim 6, wherein in the mixture, the rebaudioside A has a concentration of 1 g/L to 100 g/L; in the mixture, the sucrose has a concentration of 1 mM to 500 mM; in the mixture, the 5-uridine diphosphate disodium salt has a concentration of 0.1 mM to 20 mM; the salt is at least one of a calcium salt, a cobalt salt, an iron salt, a magnesium salt, a manganese salt, an ammonium salt, a nickel salt and a zinc salt; in the mixture, the salt has a concentration of 0.1 mM to 10 mM; the buffer solution has a pH of 5.0 to 9.0; in the stirring state, a stirring speed is 50 rpm to 220 rpm; and the enzyme-catalyzed reaction occurs at a temperature of 20°C to 45°C for a duration of 1 h to 10 h.

8. The method for efficiently preparing rebaudioside M using the biological enzyme method according to claim 7, wherein the salt is at least one of a calcium salt, a magnesium salt, a manganese salt and an iron salt; in the mixture, the salt has a concentration of 0.1 mM to 5 mM; the buffer solution has a pH of 6.0 to 8.0; and the enzyme-catalyzed reaction occurs at a temperature of 30°C to 37°C.

9. The method for efficiently preparing rebaudioside M using the biological enzyme method according to claim 6, wherein in the mixture, the glycosyltransferase has a concentration of 0.1 g/L to 20 g/L; and a sequence of the glycosyltransferase is at least one of amino acid sequences as set forth in SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11 and SEQ ID NO. 12.

10. The method for efficiently preparing rebaudioside M using the biological enzyme method according to claim 6, wherein in the mixture, the sucrose synthetase has a concentration of 0.1 g/L to 10 g/L; and a sequence of the sucrose synthetase is at least one of amino acid sequences as set forth in SEQ ID NO. 13, SEQ ID NO. 14, SEQ ID NO. 15, SEQ ID NO. 16, SEQ ID NO. 17, SEQ ID NO. 18, SEQ ID NO. 19 and SEQ ID NO. 20.
